# EUROPÄISCHE PATENTSCHRIFT

(11) **EP 2 432 602 B1**
(45) Veröffentlichungstag und Bekanntmachung des Hinweises auf die Patenterteilung: **07.06.2017**
(21) Anmeldenummer: 10721621.0
(22) Anmeldetag: 19.05.2010
(51) Int. Cl.: B21C 1/00, B21C 1/26, B21C 3/04, B21C 3/16, B21C 9/00, B21G 1/08, A61F 2/82

(54) **VERFAHREN ZUR HERSTELLUNG EINES DÜNNWANDIGEN RÖHRCHENS AUS EINER MAGNESIUMLEGIERUNG**
PROCESS FOR MANUFACTURING A SMALL DIAMETER TUBE WITH THIN WALL FROM A MAGNESIUM ALLOY
PROCÉDÉ DE FABRICATION D'UN TUBE DE PETIT DIAMÈTRE À PAROI FINE EN ALLIAGE DE MAGNÉSIUM

(30) Priorität: 19.05.2009 AT 7802009
(43) Veröffentlichungstag der Anmeldung: 28.03.2012
(73) Patentinhaber: LKR Leichtmetallkompetenzzentrum Ranshofen GmbH, 5282 Ranshofen (AT)
(72) Erfinder: BIRGMANN, Alois, A-5141 Moosdorf (AT); BRANDECKER, Martin, A-4963 St. Peter (AT); KÜHLEIN, Maria, 94166 Stubenberg (DE); WALTENBERGER, Thomas, A-5280 Braunau am Inn (AT)
(74) Vertreter: Wirnsberger, Gernot
(86) Internationale Anmeldenummer: PCT/AT2010/000171
(87) Internationale Veröffentlichungsnummer: WO 2010/132910

(56) Entgegenhaltungen:
- EP-A1- 1 338 293
- WO-A1-97/03769
- DE-A1- 2 057 865
- US-A- 1 802 785
- US-A- 2 950 816
- US-A- 3 408 846
- US-A1- 2007 077 163

## Beschreibung

Die Erfindung betrifft ein Verfahren zur Herstellung eines dünnwandigen Röhrchens für eine medizinische Anwendung oder für ein medizinisches Produkt, insbesondere für einen Stent, wobei ein Rohling aus einer insbesondere bioresorbierbaren Magnesiumlegierung zu einem Röhrchen umgeformt wird, wonach das Röhrchen medizinisch angewendet oder daraus das medizinische Produkt wie ein Stent gefertigt werden kann, wobei ein Stempel mit einem Grundkörper und einem relativ zum Grundkörper verjüngten Dorn sowie der Rohling mit einem Sackloch oder einer Durchbrechung bereitgestellt wird und wobei ein Durchmesser des Sackloches oder der Durchbrechung des Rohlings gleich oder größer als ein äußerer Durchmesser des Domes ist, wonach der Rohling bei eingeführtem Dorn mit dem Stempel zumindest teilweise durch eine Matrize mit einem Aufnahmebereich und einem Konturgebungsbereich vorwärts gepresst wird, wobei der Konturgebungsbereich einen freien Durchmesser aufweist, welcher größer als der äußere Durchmesser des Dornes, aber kleiner als ein Außendurchmesser des Rohlings ist, um das Röhrchen zu bilden. Ein Verfahren gemäß dem Oberbegriff des Anspruchs 1 ist aus der US 3 408 846 A bekannt. In der US 3 408 846 A ist ein Verfahren zur Herstellung von Nadeln bzw. Röhrchen aus einem Rohling offenbart, wobei der Rohling durch eine Matrize vorwärts gepresst wird.

Die US 2007/077163 A1 offenbart ein Verfahren zur Herstellung eines dünnwandigen Röhrchens für eine medizinische Anwendung, wobei das Röhrchen aus einer Magnesiumlegierung hergestellt wird.

In der Medizin finden sogenannte Stents Verwendung, um Blutgefäße bei medizinischem Bedarf in ihrer Form zu stützen. Ein Stent wird z. B. durch Laserschneiden aus einem Röhrchen hergestellt, sodass eine gitterartige Struktur erhalten wird, die nach Einführen in ein Blutgefäß elasto-plastisch aufgeweitet werden kann, um im Blutgefäß eine stützende Wirkung zu entfalten.

Üblicherweise wird ein Stent aus nicht rostendem Stahl oder Kunststoff hergestellt, mit dem Nachteil, dass nach Einsetzen des Stents Entzündungen oder dauerhafte Gewebereaktionen ausgelöst werden können. Zudem kann ein Stent ein Blutgefäß versteifen, wodurch es zu einer Behinderung von elastischen Bewegungen kommen kann, mit welchen ein Blutgefäß Blut optimal durch den Körper transportiert. Daher sind nach Einführen eines Stents oftmals postoperative Maßnahmen erforderlich.

Neuere Untersuchungen lassen erwarten, dass zumindest bestimmte ausgewählte Magnesiumlegierungen als Stentwerkstoffe geeignet und im menschlichen oder tierischen Körper einsetzbar sind. Magnesiumlegierungen korrodieren in Körperflüssigkeiten wie Blut sukzessive und können bei den freigesetzten Mengen je Zeiteinheit vom Körper problemlos resorbiert werden. Das heißt, dass ein Stent bereitgestellt werden kann, welcher die geforderte Stützwirkung über einen bestimmten Zeitraum erfüllt und sich schließlich ohne nachteilige Wirkungen auflöst.

Als Halbzeug für eine Herstellung von Stents werden Röhrchen eingesetzt, die mittels Rohrziehverfahren hergestellt werden. Die geometrischen Anforderungen an die Röhrchen sind definiert durch Toleranzen in Bezug auf Wanddicke, Außendurchmesser, Konzentrizität und Rundheit, die sich bei üblichen Wanddicken von 0,1 bis 1 mm und Außendurchmessern von 2 bis 20 mm in Größenordnungen von wenigen 0,001 mm bewegen. Die Länge üblicher Stents liegt etwa im Bereich von 20 bis 200 mm.

Eine Herstellung des Halbzeugs durch Rohrziehen ist sehr aufwendig, da bis zur Erzielung einer Endgeometrie mehrere Umformschritte bzw. Ziehstufen mit zwischengelagerten, mehrstündigen Wärmebehandlungen zur Wiederherstellung der Umformbarkeit des Werkstoffes erforderlich sind. Damit gehen hohe fertigungstechnische, energetische und zeitliche Aufwände einher. Beispielsweise kann ein Fertigungsprozess für Halbzeugröhrchen bis zu 60 Tage in Anspruch nehmen.

Hier setzt die Erfindung an. Aufgabe der Erfindung ist es, ein Verfahren der eingangs genannten Art anzugeben, mit dem rasch und mit hoher Fertigungsqualität dünnwandige Röhrchen aus einer Magnesiumlegierung gefertigt werden können.

Die verfahrensmäßige Aufgabe wird gelöst, wenn bei einem Verfahren der eingangs genannten Art der Rohling aus einer stranggepressten Magnesiumlegierung gebildet wird.

Ein mit der Erfindung erzielter Vorteil ist darin zu sehen, dass durch Massivumformung aus einem Rohling aus einer Magnesiumlegierung ein Röhrchen erstellt wird, sodass nur ein Verfahrensschritt notwendig ist, um eine Rohrendgeometrie zu erreichen. Darüber hinaus kann das Röhrchen mit hoher Fertigungsqualität erstellt werden, insbesondere in Bezug auf die Abmessungen des Röhrchens, wobei äußerst geringe Toleranzen eingehalten werden können, da der Konturgebungsbereich der Matrize und der äußere Durchmesser des Domes den Außendurchmesser des Röhrchens bzw. dessen freien inneren Durchmesser bestimmen. Aus einem erfindungsgemäß hergestellten Röhrchen kann ohne Weiteres beispielsweise durch Laserschneiden ein Stent erstellt werden.

Zur Durchführung des erfindungsgemäßen Verfahrens ist es ausreichend, dass der Rohling mit einem Sackloch ausgebildet ist. Zweckmäßiger ist es jedoch, dass der Rohling mit einer Durchbrechung bereitgestellt wird, deren Durchmesser dem Durchmesser des Domes entspricht oder geringfügig größer als dieser ist, sodass der Dorn durch die Durchbrechung geführt werden kann.

Der Außendurchmesser des Rohlings wird bevorzugt entsprechend einem freien Durchmesser des Aufnahmebereiches gewählt, damit beim Pressen des Rohlings derselbe im Aufnahmebereich anliegt und Material des Rohlings ausschließlich in einen freien Bereich zwischen Konturgebungsbereich und Dorn gepresst wird. Dabei erweist es sich als zweckmäßig, dass der Rohling in zylindrischer Form eingesetzt wird, da der Aufnahmebereich in der Regel ebenfalls zylindrisch ausgebildet ist.

Bevorzugt ist es, um Presskräfte möglichst gering zu halten und eine Umformung auf einfache Weise zu erreichen, dass der Rohling im erwärmten Zustand und/oder unter Erwärmung durch die Matrize gepresst wird. Diesbezüglich erweist es sich für die meisten Magnesiumlegierungen als zweckmäßig, wenn der Rohling mit einer Temperatur im Bereich von 200 °C bis 450 °C durch die Matrize gepresst wird. Bei Temperaturen von weniger als 200 °C können hohe Presskräfte erforderlich sein. Bei Temperaturen von mehr als 450 °C kann eine Magnesiumlegierung so weich sein, dass eine Verarbeitung ebenfalls erschwert ist. Im Einzelfall können aber, je nach Legierungssystem, auch höhere Temperaturen vorgesehen sein, sofern ein ausreichender Abstand zum Schmelzpunkt bzw. Schmelzintervall gehalten wird.

Insbesondere wenn der Rohling im erwärmten Zustand durch die Matrize gepresst wird oder eine Erwärmung beim Pressen erfolgen soll, ist es zweckmäßig, dass die Matrize und/oder der Stempel beheizt werden.

Es ist vorgesehen, dass der Rohling aus einer stranggepressten Magnesiumlegierung gebildet wird. Grundsätzlich kann zwar auch Gussmaterial unmittelbar eingesetzt werden, allerdings können sich dann Inhomogenitäten, die auf den Guss zurückzuführen sind, im Röhrchen finden. Dies kann insbesondere dann von Nachteil sein, wenn aus dem Röhrchen durch Laserschneiden oder auf andere Art ein Stent erstellt wird, dessen feines Gitter bei Vorliegen von Inhomogenitäten in den dünnen Stegen weniger beanspruchbar ist, was sich insbesondere beim Aufdehnen des Stents in einem Blutgefäß als nachteilig erweist. Ist der Rohling hingegen stranggepresst, so können höherwertige Stents erstellt werden, da es bei einem Strangpressen zu einer Kornfeinung und zu einer Homogenisierung des Gefüges kommt, sodass bereits ein optimales Ausgangsmaterial vorliegt, das dann nochmals gepresst wird, wobei sich eine weitere Kornfeinung und Homogenisierung des Gefüges ergibt. Darüber hinaus können dadurch die mechanischen Eigenschaften sowie die Korrosionsbeständigkeit verbessert werden. Eine Wärmebehandlung des so erstellten Röhrchens kann somit, abhängig von der verarbeiteten Legierung, mitunter sogar entfallen.

Das erfindungsgemäße Verfahren bewährt sich besonders für Röhrchen mit kleinen Außendurchmessern und geringer Wandstärke. Bevorzugt ist es, dass der Rohling mit einem Außendurchmesser von maximal 10 mm eingesetzt wird und das Röhrchen mit einem Außendurchmesser von weniger als 3 mm, vorzugsweise weniger als 2,5 mm, und einer Wandstärke von weniger als 0,5 mm, vorzugsweise weniger als 0,35 mm, insbesondere 70 bis 300 µm, erstellt wird.

Um in kurzer Zeit mehrere gleiche Röhrchen erstellen zu können, ist es zweckmäßig, dass ein am Stempel haftender Pressrest beim Zurückfahren des Stempels in eine Ausgangsposition mittels eines Abstreifers abgezogen wird. Aus dem gleichen Grund kann auch vorgesehen sein, dass nach Zurückfahren des Stempels ein Pressrest aus der Matrize ausgestoßen wird.

Ein mit einer Verwendung einer Vorrichtung erzielter Vorteil ist darin zu sehen, dass aufgrund der vorgesehenen geometrischen Ausbildung der Übergänge vom Grundkörper zum Dorn des Stempels und vom Aufnahmebereich zum Konturgebungsbereich der Matrize Presskräfte gering gehalten werden können, was eine rasche Umformung eines Rohlings begünstigt. Darüber hinaus können dünnwandige Röhrchen mit hoher Fertigungsqualität erstellt werden.

Bevorzugt ist es, dass bei einer Verwendung einer Vorrichtung zum Herstellen eines dünnwandigen Röhrchens für medizinische Anwendungen oder für ein medizinisches Produkt aus einem Rohling aus einer Magnesiumlegierung, der erste Übergang als konkaver Bogen mit einer Länge von maximal 2 mm, vorzugsweise 1,5 mm, oder als Fase mit einer solchen Länge und einem Winkel von maximal 160°, vorzugsweise maximal 130°, ausgebildet ist und der zweite Übergang als konvexer Bogen mit einer Länge von maximal 2 mm oder als Fase mit einer solchen Länge und einem Winkel von maximal 160°, vorzugsweise maximal 130°, ausgebildet ist.

Bevorzugt sind bei einer Verwendung einer Vorrichtung eine oder mehrere Heizeinrichtungen für den Stempel und/oder die Matrize vorgesehen.

Weitere Merkmale, Vorteile und Wirkungen der Erfindung ergeben sich aus der nachfolgenden Beschreibung eines Ausführungsbeispiels. In den Zeichnungen, auf welche dabei Bezug genommen wird, zeigen:
Fig. 1 eine Vorrichtung in stirnseitiger Ansicht;
Fig. 2 einen Querschnitt gemäß der Linie II-II in Fig. 1;
Fig. 3 einen vergrößerten Ausschnitt gemäß dem Kreis III in Fig. 2;
Fig. 4 eine Matrize in Draufsicht;
Fig. 5 einen Querschnitt durch eine Matrize gemäß Fig. 4;
Fig. 6 einen vergrößerten Ausschnitt entsprechend dem Kreis VI in Fig. 5;
Fig. 7 einen Stempel mit einem Bogen;
Fig. 8 einen Stempel mit einer Fase;
Fig. 9a einen Querschliff eines stranggepressten Bolzens;
Fig. 9b einen Längsschliff eines stranggepressten Bolzens;
Fig. 10a einen Querschliff eines Röhrchens;
Fig. 10b einen Längsschliff eines Röhrchens.

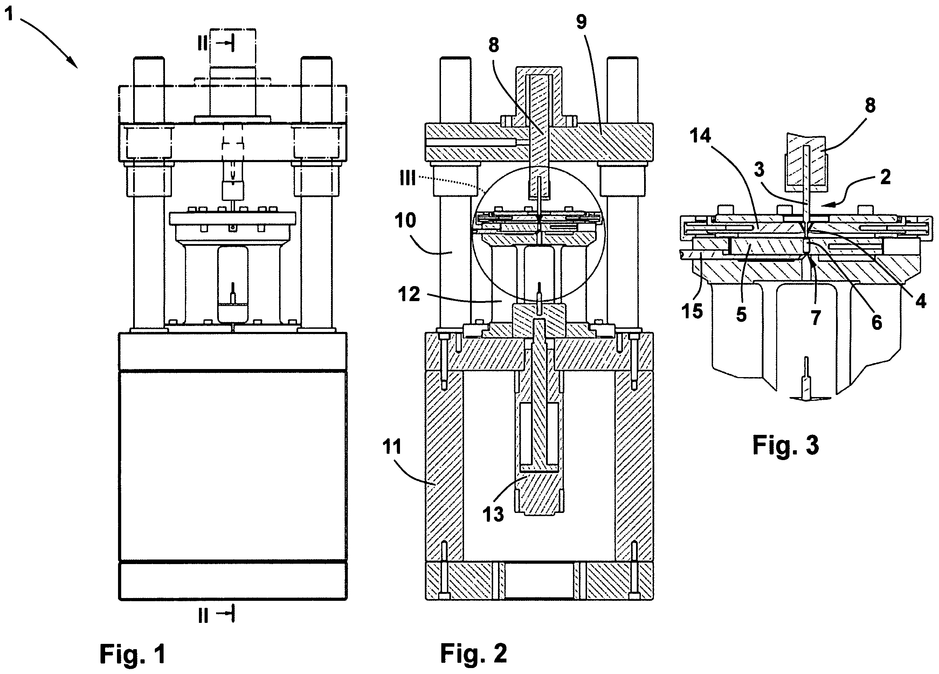

In Fig. 1 bis 3 ist eine Vorrichtung 1 dargestellt, mit welcher dünnwandige Röhrchen aus einer Magnesiumlegierung hergestellt werden können. Die Vorrichtung 1 umfasst einen Stempel 2 mit einem Grundkörper 3 und einem Dorn 4. Der Dorn 4 ist massiv ausgebildet und fest mit dem Grundkörper 3 verbunden, wenngleich der Dorn 4 auch gegenüber dem Grundkörper 3 verschiebbar gelagert sein kann. Der Stempel 2 ist im Bereich eines Endes des Grundkörpers 3 in einem Halter 8 befestigt. Der Halter 8 ist wiederum in einem waagrechten Profil 9 angeordnet, welches Profil 9 auf mehreren Führungen 10 verschiebbar gelagert ist, sodass der Halter 8 und damit auch der Stempel 2 vertikal in eine Matrize 5 verfahrbar ist.

Die Führungen 10 und die Matrize 5 sind auf einem Sockel 11 angeordnet, wobei die Matrize 5 mit Führungsschienen 12 vom Sockel 11 beabstandet gehalten ist. Die Führungsschienen 12 dienen nicht nur der Beabstandung der Matrize 5 vom Sockel 11, sondern gleichzeitig als Führung für eine Einrichtung 13, welche vertikal verfahrbar ist und mit deren Spitze, die gleiche Abmessungen aufweist wie der Dorn 4, Pressreste aus der Matrize 5 gestoßen werden können.

Wie aus Fig. 3 ersichtlich ist, ist oberhalb der Matrize 5 eine Abstreiferscheibe 14 mit einer zentralen Öffnung angeordnet. Ein Durchmesser der zentralen Öffnung entspricht einem äußeren Durchmesser des Grundkörpers 3. Dadurch kann sichergestellt werden, dass gegebenenfalls am Stempel 2 haftende Pressreste beim Zurückfahren in eine Ausgangsposition desselben abgestreift werden. Unterhalb der Matrize 5 ist eine in Grenzen waagrecht verschiebbare Abschereinrichtung 15 vorgesehen, mit welcher ein erstelltes Röhrchen vom Rest eines Rohlings bzw. Pressrest abgetrennt werden kann.

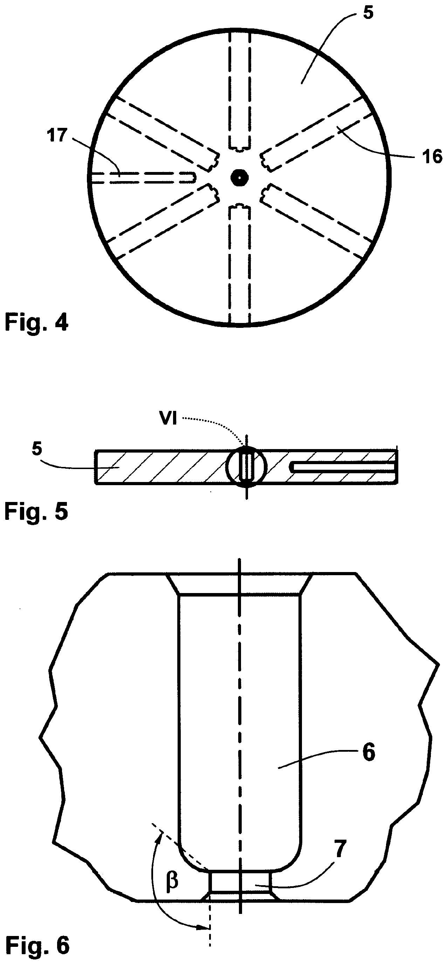

Die Matrize 5 ist in Fig. 4 bis 6 noch näher dargestellt. Die Matrize 5 ist kreisförmig ausgebildet und kann einen Durchmesser von bis zu 300 mm aufweisen. Die Matrize 5 ist mit mehreren Heizpatronen 16 bestückt, mit welchen die Matrize 5 beheizt werden kann. Des Weiteren ist ein bloß angedeutetes Gewinde 17 vorgesehen, in welches ein Justierbolzen eingreift, sodass eine Öffnung der Matrize 5 in Bezug auf eine vertikale Achse, entlang welcher der Stempel 2 bzw. eine Spitze der Einrichtung 13 verfährt, optimal justierbar ist. Im Bereich der Öffnung der Matrize 5 sind ein Aufnahmebereich 6 und ein Konturgebungsbereich 7 vorgesehen. Der Aufnahmebereich 6 weist einen freien Durchmesser auf, der dem äußeren Durchmesser des Grundkörpers 3 entspricht. Der Aufnahmebereich 6 geht im Bereich seines unteren Endes über einen konvexen Bogen in den Konturgebungsbereich 7 über. Der Bogen weist typischerweise eine Länge von weniger als 10 mm auf und kann alternativ auch durch eine Fase entsprechender Länge ersetzt sein, wobei dann ein Winkel β maximal 160° beträgt. Der Konturgebungsbereich 7 weist einen freien Durchmesser auf, der maximal 1 mm größer ist als ein äußerer Durchmesser des Domes 4, sodass sich besonders dünnwandige Röhrchen herstellen lassen.

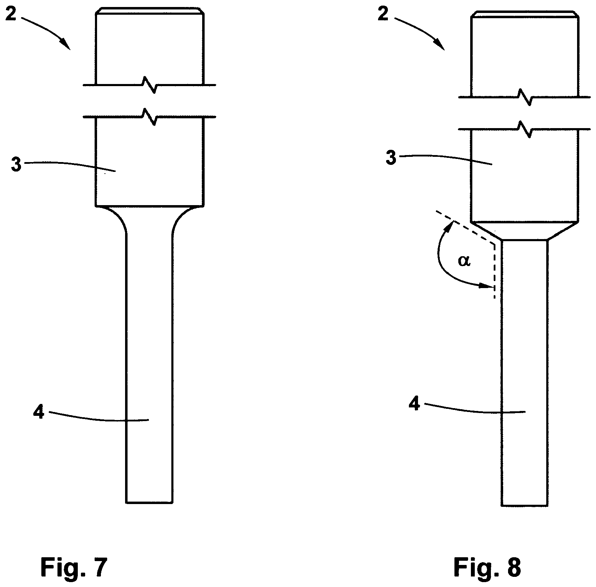

In Fig. 7 und 8 sind verschiedene Ausführungsformen des Stempels 2 gezeigt, der bei der Herstellung eines Röhrchens mit der Matrize 5 zusammenwirkt. In der Variante gemäß Fig. 7 ist ein Übergang vom Grundkörper 3 zum Dorn 4 als konkaver Bogen ausgebildet, dessen Länge kleiner als 10 mm ist. Der konkave Bogen kann gemäß Fig. 8 auch durch eine Fase entsprechender Länge ersetzt sein, wobei ein Winkel α maximal 160° beträgt.

Zur Herstellung eines dünnwandigen Röhrchens aus einer Magnesiumlegierung, insbesondere einer Magnesium-Knetlegierung, wird vorerst durch Strangpressen ein zylindrischer Bolzen erstellt, dessen äußerer Durchmesser einem freien Durchmesser des Aufnahmebereiches 6 entspricht, und ein Rohling abgelängt. Anschließend wird in den Rohling eine zentrale Durchbrechung eingebracht, beispielsweise durch Bohren. Danach wird der Rohling in den Aufnahmebereich 6 der Matrize 5 eingesetzt. Alternativ ist es auch möglich, den Rohling auf den Dorn 4 des Stempels 2 aufzusetzen. Schließlich wird der Stempel 2 in der Darstellung gemäß Fig. 2 nach unten, also in die Matrize 5 verfahren. Dabei wird auf den Rohling im Bereich des Überganges vom Grundkörper 3 zum Dorn 4 eine Presskraft aufgebracht. Da ein äußerer Durchmesser des Rohlings einem freien Durchmesser des Aufnahmebereiches 6 entspricht, kann sich der Rohling lediglich vorwärts in Richtung des Konturgebungsbereiches 7 verformen, wobei ein freier Durchmesser des Konturgebungsbereiches 7 eine äußere Form des erstellten Röhrchens definiert, wohingegen der Dorn 4 einen freien inneren Querschnitt des Röhrchens festlegt. Die Presskräfte sind bei einer erfindungsgemäßen Verwendung einer Vorrichtung insgesamt relativ gering, was auf die vorgesehenen Übergänge und eine gute Krafteinleitung in den Rohling zurückzuführen ist. Zweckmäßig ist es in den meisten Fällen auch, den eingesetzten Rohling vor und/oder während der Umformung zu erwärmen, um ein Fließen des Werkstoffes zu erleichtern. Typische Temperaturbereiche für Magnesiumlegierungen liegen im Bereich von 200 °C bis 450 °C. Grundsätzlich ist es aber auch denkbar, den Rohling durch Kaltumformung zu verformen und das so erstellte Röhrchen anschließend einer Wärmebehandlung zu unterwerfen oder durch eine ausreichend große Umformgeschwindigkeit und eine damit einhergehende Umformwärme passiv für eine Erwärmung zu sorgen. Es versteht sich, dass in der Praxis bei einer Serienfertigung mehrere Vorrichtungen 1 parallel nebeneinander laufen können, um in kurzer Zeit hohe Stückzahlen von Röhrchen zu erreichen.

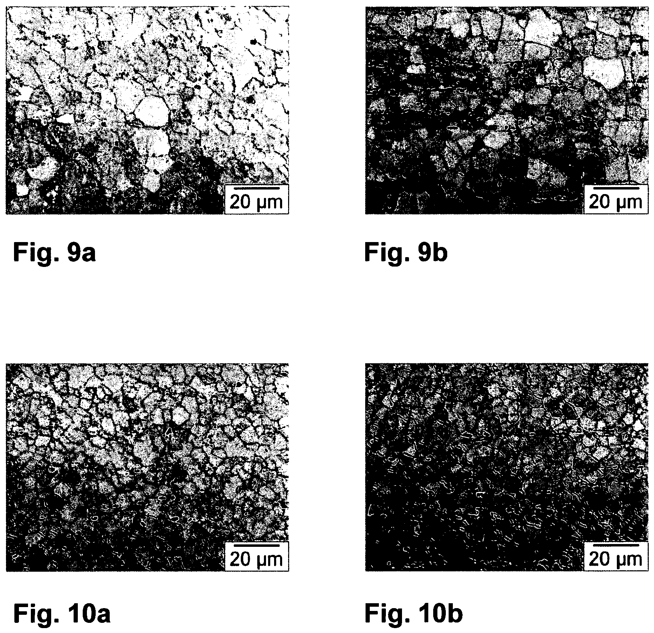

In Fig. 9a ist ein Querschliff eines stranggepressten Bolzens aus einer Magnesiumlegierung gezeigt, von dem Rohlinge zur Herstellung von Röhrchen abgenommen wurden. In Fig. 9b ist ein entsprechender Längsschliff gezeigt. Wie aus Fig. 9a und 9b ersichtlich ist, ist das Gefüge des Bolzens weitgehend homogen und weist eine durchschnittliche Korngröße von deutlich weniger als 20 µm auf.

In Fig. 10a und 10b sind ein Querschliff und ein Längsschliff eines Röhrchens gezeigt, das aus einem Rohling mit Ausgangsgefüge gemäß Fig. 9a bzw. 9b gefertigt wurde. Wie ersichtlich ist, führte die Umformung des Rohlings zur Ausbildung des Röhrchens zu einer weiteren Homogenisierung des Gefüges sowie zu einer Kornfeinung, was in Bezug auf mechanische Eigenschaften günstig ist, insbesondere wenn aus dem Röhrchen ein Stent erstellt wird, der lediglich aus dünnen, miteinander verbundenen Stegen besteht, die zudem einer Beanspruchung bei einer Ausdehnung des Stents standhalten müssen.

Durch eine Einstellung der Korngröße des Gefüges kann auch ein Korrosionsverhalten der Röhrchen bzw. daraus erstellter Stents eingestellt werden, sodass es möglich ist, Stents mit vorbestimmter Lebensdauer im menschlichen oder tierischen Körper herzustellen.

## Patentansprüche

1. Verfahren zur Herstellung eines dünnwandigen Röhrchens für eine medizinische Anwendung oder für ein medizinisches Produkt, insbesondere für einen Stent, wobei ein Rohling aus einer insbesondere bioresorbierbaren Magnesiumlegierung zu einem Röhrchen umgeformt wird, wonach das Röhrchen medizinisch angewendet oder daraus das medizinische Produkt wie ein Stent gefertigt werden kann, wobei ein Stempel (2) mit einem Grundkörper (3) und einem relativ zum Grundkörper (3) verjüngten Dorn (4) sowie der Rohling mit einem Sackloch oder einer Durchbrechung bereitgestellt wird und wobei ein Durchmesser des Sackloches oder der Durchbrechung des Rohlings gleich oder größer als ein äußerer Durchmesser des Domes (4) ist, wonach der Rohling bei eingeführtem Dorn (4) mit dem Stempel (2) zumindest teilweise durch eine Matrize (5) mit einem Aufnahmebereich (6) und einem Konturgebungsbereich (7) vorwärts gepresst wird, wobei der Konturgebungsbereich (7) einen freien Durchmesser aufweist, welcher größer als der äußere Durchmesser des Dornes (4), aber kleiner als ein Außendurchmesser des Rohlings ist, um das Röhrchen zu bilden, **dadurch gekennzeichnet, dass** der Rohling aus einer stranggepressten Magnesiumlegierung gebildet wird.

2. Verfahren nach Anspruch 1, **dadurch gekennzeichnet, dass** der Rohling mit einer Durchbrechung bereitgestellt wird, deren Durchmesser dem Durchmesser des Dornes (4) entspricht oder geringfügig größer als dieser ist.

3. Verfahren nach Anspruch 1 oder 2, **dadurch gekennzeichnet, dass** der Außendurchmesser des Rohlings entsprechend einem freien Durchmesser des Aufnahmebereiches (6) gewählt wird.

4. Verfahren nach Anspruch 3, **dadurch gekennzeichnet, dass** der Rohling in zylindrischer Form eingesetzt wird.

5. Verfahren nach einem der Ansprüche 1 bis 4, **dadurch gekennzeichnet, dass** der Rohling im erwärmten Zustand und/oder unter Erwärmung durch die Matrize (5) gepresst wird.

6. Verfahren nach Anspruch 5, **dadurch gekennzeichnet, dass** der Rohling mit einer Temperatur im Bereich von 200 °C bis 450 °C durch die Matrize (5) gepresst wird.

7. Verfahren nach einem der Ansprüche 1 bis 6, **dadurch gekennzeichnet, dass** die Matrize (5) und/oder der Stempel (2) beheizt werden.

8. Verfahren nach einem der Ansprüche 1 bis 7, **dadurch gekennzeichnet, dass** der Rohling mit einem Außendurchmesser von maximal 10 mm eingesetzt wird und das Röhrchen mit einem Außendurchmesser von weniger als 3 mm, vorzugsweise weniger als 2,5 mm, und einer Wandstärke von weniger als 0,5 mm, vorzugsweise weniger als 0,35 mm, insbesondere 70 bis 300 µm, erstellt wird.

9. Verfahren nach einem der Ansprüche 1 bis 8, **dadurch gekennzeichnet, dass** ein am Stempel (2) haftender Pressrest beim Zurückfahren des Stempels (2) in eine Ausgangsposition mittels eines Abstreifers abgezogen wird.

10. Verfahren nach Anspruch 9, **dadurch gekennzeichnet, dass** nach Zurückfahren des Stempels (2) ein Pressrest aus der Matrize (5) ausgestoßen wird.

11. Verfahren nach einem der Ansprüche 1 bis 10, **dadurch gekennzeichnet, dass** eine Vorrichtung (1) zum Herstellen eines dünnwandigen Röhrchens für medizinische Anwendungen oder für ein medizinisches Produkt aus einem Rohling aus einer Magnesiumlegierung verwendet wird, wobei ein erster Übergang vom Grundkörper (3) zum Dorn (4) als konkaver Bogen mit einer Länge von maximal 10 mm oder als Fase mit einer solchen Länge und einem Winkel (α) von maximal 160° ausgebildet ist, ein zweiter Übergang von dem Aufnahmebereich (6) zu dem Konturgebungsbereich (7) der Matrize (5) als konvexer Bogen mit einer Länge von maximal 10 mm oder als Fase mit einer solchen Länge und einem Winkel (β) von maximal 160° ausgebildet ist, ein äußerer Durchmesser des Domes (4) maximal 1 mm kleiner ist als ein freier Durchmesser des Konturgebungsbereiches (7) und ein äußerer Durchmesser des Grundkörpers (3) einem freien Durchmesser des Aufnahmebereiches (6) entspricht.

12. Verfahren nach Anspruch 11, **dadurch gekennzeichnet, dass** der erste Übergang als konkaver Bogen mit einer Länge von maximal 2 mm, vorzugsweise 1,5 mm, oder als Fase mit einer solchen Länge und einem Winkel (α) von maximal 160°, vorzugsweise maximal 130°, ausgebildet ist und der zweite Übergang als konvexer Bogen mit einer Länge von maximal 2 mm oder eine Fase mit einer solchen Länge und einem Winkel (β) von maximal 160°, vorzugsweise maximal 130°, ausgebildet ist.

13. Verfahren nach Anspruch 11 oder 12, **dadurch gekennzeichnet, dass** eine oder mehrere Heizeinrichtungen für den Stempel (2) und/oder die Matrize (5) vorgesehen sind.

## Claims

1. A method for manufacturing a small thin-walled tube for a medical application or for a medical product, in particular for a stent, wherein a blank is transformed into a tube from a particularly bio-reabsorbable magnesium alloy, whereupon the small tube may be used medically, or a medical product such as a stent may be produced therefrom, wherein a stamp (2) with a main body (3) and a mandrel (4) tapered relative to the main body (3), and the blank having a blind hole or a break-through are provided, and wherein a diameter of the blind hole or the break-through of the blank is equal to or greater than an external diameter of the mandrel (4), whereupon the blank, with the mandrel (4) inserted, is at least partially pressed forward, with the stamp (2), through a die (5) which has a receiving area (6) and a contour-defining area (7), wherein the contour-defining area (7) has a free diameter, which is larger than the external diameter of the mandrel (4), but smaller than an external diameter of the blank, in order to form the small tube, **characterised in that** the blank is formed of an extruded magnesium alloy.

2. The method according to claim 1, **characterised in that** the blank is provided with a break-through, the diameter of which corresponds to, or is slightly larger than, the diameter of the mandrel (4).

3. The method according to claim 1 or 2, **characterised in that** the external diameter of the blank is selected according to a free diameter of the receiving area (6).

4. The method according to claim 3, **characterised in that** the blank is deployed in a cylindrical form.

5. The method according to one of claims 1 to 4, **characterised in that** the blank is pressed through the die (5) in a heated state, and/or whilst being heated.

6. The method according to claim 5, **characterised in that** the blank is pressed through the die (5) at a temperature in the range between 200°C to 450°C.

7. The method according to one of claims 1 to 6, **characterised in that** the die (5) and/or the stamp (2) are heated.

8. The method according to one of claims 1 to 7, **characterised in that** the blank is deployed with an external diameter of maximum 10 mm and the small tube is created with an external diameter of less than 3 mm, preferably less than 2.5 mm, and a wall thickness of less than 0.5 mm, preferably less than 0.35 mm, in particular 70 to 300 µm.

9. The method according to one of claims 1 to 8, **characterised in that** a pressing residue adhering to the stamp (2) is removed by means of a scraper as the stamp (2) retracts into a starting position.

10. The method according to claim 9, **characterised in that** following retraction of the stamp (2) a pressing residue is expelled from the die (5).

11. The method according to one of claims 1 to 10, **characterised in that** a device (1) for manufacturing a small thin-walled tube for medical applications or for a medical product from a blank made of a magnesium alloy is used, wherein a first transition from the main body (3) to the mandrel (4) is configured as a concave arc with a maximum length of 10 mm or as a chamfer of that length and with a maximum angle (α) of 160°, a second transition from the receiving area (6) to the contour-defining area (7) of the die (5) is configured as a convex arc with a maximum length of 10 mm or as a chamfer of that length and with a maximum angle (β) of 160°, an external diameter of the mandrel (4) is smaller by a maximum of 1 mm than a free diameter of the contour-defining area (7) and an external diameter of the main body (3) corresponds to a free diameter of the receiving area (6).

12. The method according to claim 11, **characterised in that** the first transition is configured as a concave arc with a maximum length of 2 mm, preferably 1.5 mm, or as a chamfer of that length and with a maximum angle (α) of 160°, preferably a maximum angle of 130°, and the second transition is configured as a convex arc with a maximum length of 2 mm, or a chamfer of that length and with a maximum angle (β) of 160°, preferably a maximum angle of 130°.

13. The method according to one of claims 11 or 12, **characterised in that** one or more heating units are provided for the stamp (2) and/or the die (5).

## Revendications

1. Procédé pour la fabrication de tuyaux à paroi mince pour un usage médical ou pour un produit médical, notamment pour un stent, un élément d'ébauche étant déformé en un tube capillaire à partir d'un alliage de magnésium notamment biorésorbable, selon lequel le tube capillaire peut être médicalement utilisé ou le produit médical comme un stent peut être fabriqué à partir de celui-ci, un poinçon (2) avec un corps de base (3) et une broche (4) réduite par rapport au corps de base (3) ainsi que l'élément d'ébauche avec un trou borgne ou une ouverture de passage étant préparés et un diamètre du trou borgne ou de l'ouverture de passage de l'élément d'ébauche étant égal ou supérieur à un diamètre extérieur du mandrin (4), selon lequel l'élément d'ébauche avec le mandrin(4) étant introduit est pressé en avant avec le poinçon (2) au moins en partie à travers une matrice (5) avec une zone de réception (6) et une zone de formation de contour (7), la zone de formation de contour (7) comportant un diamètre libre, lequel est supérieur au diamètre extérieur de la broche (4), mais inférieur à un diamètre extérieur de l'élément d'ébauche pour former le tube capillaire, **caractérisé en ce que** l'élément d'ébauche est formé à partir d'un alliage de magnésium extrudé.

2. Procédé selon la revendication 1, **caractérisé en ce que** l'élément d'ébauche est préparé avec une ouverture de passage, dont le diamètre correspond au diamètre du mandrin (4) ou est légèrement plus grand que celui-ci.

3. Procédé selon la revendication 1 ou 2, **caractérisé en ce que** le diamètre extérieur de l'élément d'ébauche est choisi correspondant à un diamètre libre de la zone de réception (6).

4. Procédé selon la revendication 3, **caractérisé en ce que** l'élément d'ébauche est mis en oeuvre dans une forme cylindrique.

5. Procédé selon l'une quelconque des revendications 1 à 4, **caractérisé en ce que** l'élément d'ébauche est pressé à travers la matrice (5) à l'état chaud et/ou sous réchauffement.

6. Procédé selon la revendication 5, **caractérisé en ce que** l'élément d'ébauche est pressé à travers la matrice (5) à une température se situant dans la gamme de 200°C à 450° C.

7. Procédé selon l'une quelconque des revendications 1 à 6, **caractérisé en ce que** la matrice (5) et/ou le poinçon (2) sont chauffés.

8. Procédé selon l'une quelconque des revendications 1 à 7, **caractérisé en ce que** l'élément d'ébauche est mis en oeuvre avec un diamètre extérieur de 10 mm maximum et le tube capillaire est élaboré avec un diamètre extérieur inférieur à 3 mm, de préférence inférieur à 2,5 mm et une épaisseur de paroi inférieure à 0,5 mm, de préférence inférieur à 0,35 mm, notamment de 70 à 300 µm.

9. Procédé selon l'une quelconque des revendications 1 à 8, **caractérisé en ce qu'**un résidu de pressage adhérant au poinçon (2) est retiré au moyen d'un racloir lors du recul du poinçon (2) dans une position initiale.

10. Procédé selon la revendication 9, **caractérisé en ce qu'**après recul du poinçon (2), un résidu de pressage est expulsé de la matrice (5).

11. Procédé selon l'une quelconque des revendications 1 à 10, **caractérisé en ce qu'**un dispositif (1) est utilisé pour fabriquer un tube capillaire à paroi mince à partir d'un élément d'ébauche dans un alliage de magnésium destiné à des applications médicales ou pour un produit médical, un premier passage du corps de base (3) au mandrin (4) étant constitué sous la forme d'un arc concave avec une longueur de 10 mm maximum ou sous la forme d'un chanfrein avec une longueur identique et un angle (α) de 160° maximum, un deuxième passage de la zone de réception (6) à la zone de formation de contour (7) de la matrice (5) étant constitué sous la forme d'un arc convexe avec une longueur de 10 mm maximum ou sous la forme d'un chanfrein avec une longueur identique et un angle (β) de 160° maximum, un diamètre extérieur du mandrin (4) étant au maximum de 1 mm inférieur à un diamètre libre de la zone de formation de contour (7) et un diamètre extérieur du corps de base (3) correspondant à un diamètre libre de la zone de réception (6).

12. Procédé selon la revendication 11, **caractérisé en ce que** le premier passage est constitué sous la forme d'un arc concave avec une longueur de 2 mm maximum, de préférence 1,5 mm, ou sous la forme d'un chanfrein avec une longueur identique et un angle (α) de 160° maximum, de préférence 130° maximum et le deuxième passage étant constitué sous la forme d'un arc convexe avec une longueur de 2 mm maximum ou d'un chanfrein avec une longueur identique et un angle (β) de 160° maximum, de préférence 130° maximum.

13. Procédé selon la revendication 11 ou 12, **caractérisé en ce qu'**un ou plusieurs dispositifs de chauffage sont prévus pour le poinçon (2) et/ou la matrice (5).
